(19) Europäisches Patentamt
European Patent Office
Office européen des brevets

(11) EP 2 207 422 B1

(12) # FASCICULE DE BREVET EUROPEEN

(45) Date de publication et mention de la délivrance du brevet:
**13.09.2017 Bulletin 2017/37**

(21) Numéro de dépôt: **08869457.5**

(22) Date de dépôt: **10.10.2008**

(51) Int Cl.:
***A01N 43/40*** (2006.01) ***A61K 31/44*** (2006.01)
***A61K 31/415*** (2006.01) ***A61P 33/14*** (2006.01)

(86) Numéro de dépôt international:
**PCT/FR2008/001423**

(87) Numéro de publication internationale:
**WO 2009/087286 (16.07.2009 Gazette 2009/29)**

(54) **UTILISATION D'UN DERIVE DE 1-N-(HALO-3-PYRIDYLMETHYLE)-N-METHYLAMINO-1-ALKYLAMINO-2-NITROETHYLENE POUR LA PREPARATION D'UNE COMPOSITION PHARMACEUTIQUE VETERINAIRE TOPIQUE POUR LUTTER CONTRE LES PARASITES EXTERNES**

VERWENDUNG EINES 1-N-(HALOGEN-3-PYRIDYLMETHYL)-N-METHYLAMINO-1-ALKYLAMINO-2-NITROETHYLENDERIVATS ZUR HERSTELLUNG EINER TOPISCHEN VETERINÄRPHARMAZEUTISCHEN ZUSAMMENSETZUNG FÜR DIE BEKÄMPFUNG VON EKTOPARASITEN

USE OF A 1-N-(HALO-3-PYRIDYLMETHYL)-N-METHYLAMINO-1-ALKYLAMINO-2-NITROETHYLENE DERIVATIVE FOR PREPARING A TOPICAL VETERINARY PHARMACEUTICAL COMPOSITION FOR COMBATING EXTERNAL PARASITES

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **11.10.2007 FR 0707127**

(43) Date de publication de la demande:
**21.07.2010 Bulletin 2010/29**

(73) Titulaire: **VIRBAC**
**06516 Carros (FR)**

(72) Inventeurs:
- **DERRIEU, Guy**
**F-06800 Cagnes sur Mer (FR)**
- **MARC, Jean Pascal**
**F-06570 Saint Paul (FR)**

(74) Mandataire: **Gevers & Orès**
**41 avenue de Friedland**
**75008 Paris (FR)**

(56) Documents cités:
**EP-A- 0 976 328** **EP-A- 1 668 984**
**WO-A-00/29378** **WO-A-93/24002**
**WO-A-2004/064522** **WO-A-2004/089239**
**DE-A1- 19 807 633** **GB-A- 2 321 012**

- **DATABASE WPI Week 200355 Thomson Scientific, London, GB; AN 2003-581020 XP002478383 & JP 2003 095813 A (JUKA LIFETEK KK) 3 avril 2003 (2003-04-03)**
- **DATABASE WPI Week 200337 Thomson Scientific, London, GB; AN 2003-385020 XP002478384 & JP 2003 026603 A (JUKA LIFETEK KK) 29 janvier 2003 (2003-01-29)**
- **BULLETIN OFFICIEL N°2003-21: "Avis relatif à l'octroi d'autorisations de mise sur le marché de médicaments vétérinaires"[Online] 21 mai 2003 (2003-05-21), pages 1-2, XP002478382 Extrait de l'Internet: URL:http://www.sante.gouv.fr/adm/dagpb/bo/2003/03-21/a0211447.htm> [extrait le 2008-04-28]**
- **SNYDER ET AL: "Classification of the solvent properties of common liquids", JOURNAL OF CHROMATOGRAPHY, ELSEVIER SCIENCE PUBLISHERS B.V, NL, vol. 92, no. 2, 22 May 1974 (1974-05-22), pages 223-230, XP026527138, ISSN: 0021-9673, DOI: 10.1016/S0021-9673(00)85732-5 [retrieved on 1974-05-22]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente Invention se rapporte à l'utilisation, à titre de principe actif, d'un dérivé de 1-N-(halo-3-pyridylméthyle)-N-méthylamino-1-alkylamino-2-nitroéthylène pour la préparation d'une composition pharmaceutique vétérinaire à application topique pour le traitement des infestations par des parasites externes, en particulier par les puces, chez les animaux domestiques, en particulier chez les chiens et les chats.

L'infestation des animaux domestiques par des parasites se nourrissant de sang, en particulier par des tiques et des puces, est un important problème sanitaire.

Les puces sont des insectes dépourvus d'ailes, au corps comprimé latéralement et aux pattes très développées, adaptées au saut. Ce sont des ectoparasites, suceurs de sang de mammifères ou d'oiseaux. Les quelques 2000 espèces répertoriées appartiennent à l'ordre des siphonaptères. Deux espèces de puces sont communément rencontrées en Europe ; il s'agit de la puce du chat (*Ctenocephalides felis*) et de la puce du chien (*Ctenocephalides canis*) qui vivent dans la fourrure des animaux. La puce du chat, la plus fréquente, est capable de se reproduire à la fois sur le chat et le chien. Elle peut aussi s'attaquer à l'homme et aux autres animaux de compagnie, cependant le chat est le principal responsable d'infestation lorsque chats et chiens vivent dans le même environnement.

Les puces ont un cycle de vie complexe avec 4 stades distincts : oeuf, larve, nymphe et adulte. Elles s'accouplent dans les premières 8 à 48 heures suivant l'acquisition par l'hôte, après leur premier repas sanguin. Les femelles commencent ainsi à pondre 24 à 48 heures après ce premier repas sanguin. La puce adulte pond généralement sur l'animal. Les oeufs pondus sur l'animal n'y restent d'ailleurs pas et tombent sur le sol. En conditions optimales, la femelle peut pondre plus de 25 oeufs par jour. Elle en pondra plusieurs centaines durant toute sa vie. Après quelques jours, naît une larve vermiforme, blanche et poilue, d'environ 1,5 mm de long. La larve se nourrit de débris organiques, de dépouilles larvaires et du sang sec déféqué par les adultes. L'état larvaire dure de 1 à 3 semaines, si les conditions sont favorables (18°C à 27°C et 70% d'humidité relative). La larve tisse ensuite un cocon et se nymphose. Normalement, la nymphe évolue en 1 à 2 semaines mais le passage à l'état adulte peut se prolonger jusqu'à 1 an, si les conditions sont défavorables. La puce adulte (petite et noire) émerge du cocon quand elle décèle vibrations, chaleur, concentration plus élevée en gaz carbonique, ce qui se produit lors du passage d'un chat, d'un chien... ou d'un homme ! Elle saute alors sur la victime, se nourrit aussitôt de sang et grossit rapidement en prenant une couleur plus claire d'un brun rougeâtre. La puce adulte vit de 6 à 12 mois. Sans nourriture, elle peut survivre jusqu'à 2 mois.

Les piqûres de puces provoquent des démangeaisons, chez l'animal comme chez l'homme. La salive de la puce (sécrétée à chaque piqûre) peut aussi, selon les individus, entraîner des réactions allergiques, immédiates ou retardées. Ces réactions se traduisent par diverses lésions cutanées et démangeaisons. On distingue deux types de dermatoses liées aux puces, à savoir la pulicose et la dermatite par allergie aux piqûres de puces. Si dans les deux cas, la dermatose résulte d'une infestation plus ou moins importante par des puces, ce n'est que dans la seconde qu'un phénomène allergique est associé. La dermatite par allergie aux piqûres de puces (DAPP) est la cause la plus fréquente de prurit chez le chien. En France, chez le chien adulte, elle représente ainsi près de la moitié des dermatoses prurigineuses. Près de 80 % des chiens qui présentent une DAPP ont également une dermatite atopique. Réciproquement, deux chiens atopiques sur trois présentent une DAPP. Il est donc probable que les chiens atopiques soient prédisposés au développement d'une allergie aux piqûres de puces et que l'infestation par celles-ci soit un facteur déclenchant d'une dermatite atopique. Cela justifie de la nécessité d'un contrôle antipuce très poussé chez les chiens atopiques ou appartenant à des races à risque. De plus, la DAPP est probablement la principale cause de réapparition du prurit chez les chiens atopiques désensibilisés.

Les puces du genre *Ctenocephalides* sont par ailleurs des hôtes intermédiaires de *Dipylidium caninum,* qui est un ver parasite de l'intestin grêle du chien et du chat. Le carnivore s'infeste en avalant les puces parasitées. Cette infestation peut provoquer un prurit anal, un engorgement des sacs anaux, ainsi qu'une dermatite de la région périnéale. C'est pourquoi, il est parfois conseillé de vermifuger régulièrement les animaux en complément de la lutte contre les puces.

Les infestations par les puces représentent donc un important problème pour les animaux qui sont infestés et imposent de pouvoir disposer de traitements adaptés. Il convient en particulier que le traitement ait non seulement une efficacité immédiate (rapidité d'action) mais également une efficacité prolongée dans le temps (rémanence) afin d'éviter, d'une part, les traitements répétés et, d'autre part, tout risque d'infestation et/ou de réinfestation pendant une période prolongée. La puce doit être éliminée avant qu'elle ne se reproduise et commence à pondre.

Il existe actuellement sur le marché différents types de traitements insecticides contre les puces (shampooings, poudres, aérosols, colliers, comprimés, compositions liquides à application topiques). L'ensemble de ces traitements ne donne cependant pas toujours entière satisfaction.

Les shampooings antiparasitaires constituent un bon "traitement d'attaque" lors d'infestations massives mais n'ont pas d'effet durable. Ils ne traitent que les puces adultes et sont particulièrement difficiles à utiliser sur les chats qui n'apprécient généralement pas le bain.

Les poudres antiparasitaires contiennent un principe actif insecticide dilué dans du talc. Ce mode d'application n'est pas très efficace car le talc glisse le long du pelage et le produit pénètre rarement jusqu'à la peau. De plus, les

animaux risquent d'ingérer accidentellement le principe actif insecticide ou les autres constituants de la composition en se léchant. L'emploi de poudre n'est pas non plus d'une grande commodité, dans la mesure où son application peut prendre plusieurs minutes, risque de ne pas être uniforme sur l'ensemble de la fourrure de l'animal et s'accompagne d'un dégagement de particules pulvérulentes qui sont inévitablement inhalées par l'animal et son propriétaire même en cas d'application particulièrement méticuleuse.

Ce genre de problème est également rencontré avec l'utilisation de vaporisateurs ou pulvérisateurs mécaniques, du type "spray", ou d'aérosols (par exemple à base de perméthrine à titre de principe actif) qui présentent, de plus, l'inconvénient supplémentaire de générer un stress ou une réaction agressive chez l'animal sur lequel il est appliqué compte tenu du bruit généré au moment de la pulvérisation. Les compositions appliquées grâce à ces dispositifs sont par ailleurs généralement peu rémanentes (1 semaine maximum) et résistent mal à l'eau.

Les colliers anti-puces sont généralement assez efficaces de façon temporaire. Cependant, leur efficacité est essentiellement limitée aux régions de l'animal qui sont proches du collier (cou, nez, thorax). Par contre, sur les autres parties de l'animal, l'efficacité des colliers anti-puces peut être très faible, voire inexistante. De plus, les chats qui ont l'habitude de sortir risquent d'accrocher leur collier dans une branche ou un grillage et de se faire mal en s'étranglant.

L'administration de compositions pharmaceutiques par voie systémique, notamment par voie orale ou parentérale, représente déjà un certain avancement, en termes d'efficacité, par rapport aux différents traitements détaillés ci-dessus. On trouve actuellement sur le marché un choix assez large de préparations utilisant différents principes actifs connus pour avoir une activité anti-puces. Lors d'une administration par voie orale, ces principes actifs sont absorbés au niveau digestif et se retrouvent dans le sang de l'animal pour causer l'empoisonnement des puces lors de la piqûre. Parmi ce type de produits, on peut notamment citer le produit Capstar®, utilisant comme principe actif le nitenpyram (dont le nom chimique est (E)-N-(6-chloro-3-pyridylméthyl)-N-éthyl-N'-méthyl-2-nitrovinylidéne-diamine selon la nomenclature IUPAC), comme décrit par exemple dans la demande de brevet EP 0 616 494. Le produit Capstar®, qui se présente sous la forme de comprimés pour chiens et chats, possède une rapidité d'action importante. En effet, la concentration sanguine maximale est atteinte en 30 à 120 minutes chez le chien et le chat à jeun. Cependant, la demi-vie plasmatique du principe actif est de 4 heures chez le chien et de 8 heures chez le chat ; 90 % de la molécule étant excrétés dans les urines en une journée chez le chien et en deux jours chez le chat. Ainsi l'efficacité de ce produit est très courte et il est nécessaire de l'administrer quotidiennement selon les recommandations de l'exploitant. Ce produit présente, en outre, l'inconvénient d'être sous la forme de comprimés dont l'administration n'est pas aisée, voir impossible, chez certains animaux peu coopératifs, ou particulièrement sauvages.

Enfin, les produits commercialisés actifs contre les puces peuvent également se présenter sous la forme de compositions liquides (pipettes) ou solutions cutanées pour dépôt ou encore "Spot-On", à appliquer très facilement, en une seule fois, de façon topique directement sur la peau de l'animal, généralement entre les omoplates. A titre d'exemples de ce type de produits, on peut citer :

- les produits à base de fipronil tel que le produit Frontline® Spot-On Chat et Chien, vendu en France par la société Merial SAS. Il est souvent présenté comme un produit de choix pour la prévention et le traitement des infestations par les puces. Cependant, selon les données de l'exploitant, le seuil des concentrations actives de Fipronil est dépassé au bout de 24 heures sur l'ensemble de la surface corporelle de l'animal et il présente donc l'inconvénient de ne pas être très rapidement actif. Par ailleurs, la durée de protection contre les nouvelles infestations est limitée à 4 semaines et le produit ne dispose donc pas d'une rémanence importante ;
- les produits à base d'imidaclopride tels que les produits Advantage® Chat et Advantage® Chien, vendus en France par Bayer Pharma Division Santé Animale. Selon les données de l'exploitant, les puces sont tuées dans les 24 heures après application du traitement et la durée de protection contre les nouvelles infestations est de 4 semaines. Aussi, la rapidité d'action ainsi que la rémanence de ces produits ne sont pas entièrement satisfaisantes.
- l'association de perméthrine et d'imidaclopride vendue sous la dénomination Advantix® par la société Bayer ou bien encore l'association de perméthrine et de pyriproxyfène vendue sous la dénomination Duowin® Contact par la société Virbac, qui présentent toutes deux l'inconvénient majeur d'être uniquement destinées aux chiens car elles sont très toxiques chez le chat.

D'autres documents envisagent l'administration par voie topique de produits contre les parasites externes.

En particulier, la Demande de Brevet EP 0 616 494 -citée plus haut-mentionne la voie topique parmi différentes voies d'administration des dérivés de 1-N-(halo-3-pyridylméthyle)-N-méthylamino-1-alkylamino-2-nitroéthylène ; il ne s'agit cependant pas de la voie décrite comme la plus intéressante.

La Demande de Brevet DE 198 07 633 propose une composition pour limiter le développement dermique d'insectes parasitaires chez les animaux ; cette composition à base d'eau comprend en outre un agoniste ou un antagoniste des récepteurs nicotiniques à l'acétylcholine et un solvant choisi parmi les carbonates cycliques et les lactones.

La Demande de Brevet EP 0 976 328 décrit un agent de lutte contre les ectoparasites chez les animaux comprenant un néonicotinoïde et un solvant choisi parmi un éther de glycol ou un monoalkyléther de glycol.

La Demande de Brevet WO 2004/064522 décrit des formulations topiques associant (i) un pyréthroïde de type I ou de type II, un pyréthroïde sans fonction ester ou un pyréthroïde naturel avec (ii) un composé choisi parmi les néonicotinoïdes, la nithiazine et les spinosynes.

La Demande de Brevet EP 1 668 984 décrit une association synergique, pour la voie orale ou topique, composée d'un dérivé de 1-phényl-pyrazole avec un pesticide choisi parmi les lactones macrolides, les néonicotinoïdes, les régulateurs de la croissance des insectes (IGR), les pyréthroïdes, les pyrimidines, les organophosphates et l'amitraz.

La Demande de Brevet JP 2001/0291535 décrit une composition liquide pour traiter les ectoparasites chez les chiens et les chats comprenant un pesticide, un antioxydant et un solvant huileux ; ledit pesticide étant choisi parmi les pyréthroïdes, les néonicotinoïdes, les dérivés de phényl-pyrazole, les dérivés de phényl-pyrrole, les organophosphates et les régulateurs de la croissance des insectes.

La Demande de Brevet JP 2001/0216111 décrit une composition pour traiter les ectoparasites des animaux de compagnie qui se présente sous la forme d'une suspension d'un pesticide insoluble dans de l'eau ou d'une solution d'un pesticide soluble dans l'eau. Les pesticides peuvent être choisis parmi les pyréthroïdes, les néonicotinoïdes, les dérivés de phényl-pyrazole, les dérivés de phényl-pyrrole les organophosphates, les régulateurs de la croissance des insectes et les inhibiteurs de la synthèse de chitine.

Aucun des documents qui précèdent ne met en évidence un avantage à l'administration topique du nitenpyram par rapport à d'autres voies d'administration.

La Demande de Brevet WO 00/29378, dont l'objet se rapporte à de nouveaux composés antiparasitaires, souligne même que les composés de formule générale (II) qui inclut le nitenpyram, lorsqu'ils sont administrés par voie topique, présentent une efficacité qui s'estompe rapidement après l'administration obligeant ainsi à procéder à des applications rapprochées. Il n'est toutefois pas possible à partir de ce document de savoir à quelles conditions précises de formulation et d'administration des composés de formule générale (II) correspondent ces observations.

C'est donc afin de remédier à l'ensemble des problèmes rencontrés avec les produits antiparasitaires externes, et en particulier avec les produits anti-puces actuellement disponibles sur le marché et de pourvoir à un produit permettant de prévenir et de traiter efficacement les infestations par les puces chez les animaux domestiques, aussi bien chez le chat que chez le chien que les Inventeurs ont mis au point ce qui fait l'objet de l'Invention. Ils se sont en particulier donnés pour but de pourvoir à un produit pour la prévention et le traitement des infestations par les puces chez les animaux domestiques qui soit compatible avec les différents cycles de vie des puces, facile à administrer, tout en ayant une action très rapide et plus rémanente que les produits actuellement disponibles sur le marché.

A cette occasion, la Demanderesse a découvert de façon surprenante, que le nitenpyram connu comme principe actif à administrer préférentiellement par voie systémique et notamment par voie orale, pouvait avantageusement être administré par voie topique dans une formulation liquide avec un support liquide pharmaceutiquement acceptable consistant en du monoéthylène du diéthylène glycol et que, dans cette voie d'administration, il présentait une plus grande rapidité d'action et une très bonne rémanence ; la rémanence observée lors de l'administration topique du nitenpyram est inattendue et bien meilleure que les produits à administration par voie topique de référence à base de fipronil et permet ainsi une administration plus espacée de ces dérivés.

L'objet de la présente invention est défini par les revendications 1 à 8.

La présente invention a pour objet (E)-N-(6-chloro-3-pyridylméthyl)-N-éthyl-N'-méthyl-2-nitrovinylidènediamine (nitenpyram) pour son utilisation pour la prévention et/ou le traitement des infestations par les puces chez les animaux domestiques,

caractérisé en ce que ledit nitenpyram est formulé, dans une composition pharmaceutique liquide à administrer par voie topique, avec un support liquide pharmaceutiquement acceptable consistant en du monoéthyléther du diéthylène glycol, et en ce que ladite composition est appliquée au moins toutes les 4 semaines, intervalle de temps pendant lequel l'animal n'est pas retraité.

Dans un mode de réalisation, l'invention a pour objet (E)-N-(6-chloro-3-pyridylméthyl)-N-éthyl-N'-méthyl-2-nitrovinylidènediamine (nitenpyram) tel que défini précédemment pour son utilisation telle que définie précédemment, caractérisé par le fait que ladite composition pharmaceutique est administrée chez le chat ou le chien.

Dans un mode de réalisation, l'invention a pour objet (E)-N-(6-chloro-3-pyridylméthyl)-N-éthyl-N'-méthyl-2-nitrovinylidènediamine (nitenpyram) tel que défini précédemment pour son utilisation telle que définie précédemment, caractérisé par le fait qu'au sein de ladite composition pharmaceutique, le nitenpryram représente de 5 à 20% en poids par rapport au poids total de la composition.

Dans un mode de réalisation, l'invention a pour objet (E)-N-(6-chloro-3-pyridylméthyl)-N-éthyl-N'-méthyl-2-nitrovinylidènediamine (nitenpyram) tel que défini précédemment pour son utilisation telle que définie précédemment, caractérisé par le fait que ladite composition pharmaceutique renferme en outre un ou plusieurs excipients choisis parmi les agents tensioactifs, les agents épaississants, les régulateurs de pH, les colorants, les inhibiteurs de cristallisation, les agents filmogènes, les antioxydants, et leurs mélanges.

Dans un mode de réalisation, l'invention a pour objet (E)-N-(6-chloro-3-pyridylméthyl)-N-éthyl-N'-méthyl-2-nitrovinylidènediamine (nitenpyram) tel que défini précédemment pour son utilisation telle que définie précédemment,

caractérisé par le fait que ladite composition pharmaceutique comprend également un ou plusieurs composés actifs additionnels choisis parmi les principes actifs vis-à-vis de la prévention et/ou du traitement des puces et/ou des tiques et/ou des phlébotomes chez les animaux domestiques.

Dans un mode de réalisation, l'invention a pour objet (E)-N-(6-chloro-3-pyridylméthyl)-N-éthyl-N'-méthyl-2-nitrovinylidènediamine (nitenpyram) tel que défini à l'objet précédent pour son utilisation telle que définie à l'objet précédent, caractérisé par le fait que ladite composition pharmaceutique renferme une association de (E)-N-(6-chloro-3-pyridylméthyl)-N-éthyl-N'-méthyl-2-nitrovinylidènediamine (nitenpyram) et de 5-amino-1-[2,6-dichloro-4-(trifluorométhyl)phényl]-4-(trifluorométhylsulfinyl)-1H-pyrazole-3-carbonitrile (fipronil).

Dans un mode de réalisation, l'invention a pour objet (E)-N-(6-chloro-3-pyridylméthyl)-N-éthyl-N'-méthyl-2-nitrovinylidènediamine (nitenpyram) tel que défini aux quatres premiers objets précédents pour son utilisation telle que définie aux quatres premiers objets précédents, caractérisé par le fait que ladite composition pharmaceutique comprend également un ou plusieurs composés actifs additionnels choisis parmi les principes actifs vis-à-vis de la prévention et/ou du traitement des endoparasites chez les animaux domestiques.

Dans un mode de réalisation, l'invention a pour objet (E)-N-(6-chloro-3-pyridylméthyl)-N-éthyl-N'-méthyl-2-nitrovinylidènediamine (nitenpyram) tel que défini à l'objet précédent pour son utilisation telle que définie à l'objet précédent, caractérisée par le fait que ladite composition pharmaceutique renferme une association de Nitenpyram, de Moxidectine et de Praziquantel.

La présente description a pour objet l'utilisation, à titre de principe actif, d'au moins un dérivé de 1-N-(halo-3-pyridylméthyle)-N-méthylamino-1-alkylamino-2-nitroéthylène de formule (I) suivante :

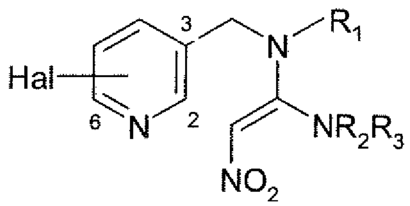

(I)

dans laquelle :

- Hal représente un atome d'halogène choisi parmi le fluor, le chlore, le brome et l'iode,
- $R_1$ et $R_2$, identiques ou différents, représentent un atome d'hydrogène, un radical alkyle en $C_1$-$C_6$ ou un radical cyclo alkyle en $C_3$-$C_7$,
- $R_3$ représente un atome d'hydrogène ou un radical alkyle en $C_1$-$C_6$, et d'au moins un support pharmaceutiquement acceptable,

pour la préparation d'une composition pharmaceutique à administrer par voie topique pour la prévention et/ou le traitement des infestations par les parasites externes, en particulier les puces, chez les animaux domestiques,
laquelle utilisation étant caractérisée en ce que ladite composition est destinée à être appliquée au moins toutes les 2 semaines, de préférence au moins toutes les 4 semaines et, encore préférentiellement, au moins toutes les 7 semaines.

Lorsque la composition est destinée à être appliquée au moins toutes les 2 semaines, on entend qu'elle permettra d'assurer une efficacité pendant au moins 14 jours et qu'il ne sera pas nécessaire de retraiter l'animal pendant cet intervalle de temps.

Selon une variante particulière de mise en oeuvre de l'utilisation selon la description, ladite composition pharmaceutique est destinée à être appliquée toutes les 2 à 12 semaines, toutes les 4 à 10 semaines ou toutes les 7 à 8 semaines.

Selon une variante particulièrement préférée, ladite composition est une composition liquide comprenant au moins un support liquide pharmaceutiquement acceptable.

Selon la description, ladite composition pharmaceutique est en particulier destinée à être administrée chez le chat ou chez le chien.

Selon cette utilisation, le ou les composés de formule (I) sont de préférence choisis parmi les composés dans lesquels l'atome d'halogène désigné par Hal est situé en position 6 du cycle pyridyle et désigne de préférence un atome de chlore.

Selon une forme de réalisation particulièrement préférée de la description, le ou les composés de formule (I) sont choisis parmi ceux dans lesquels $R_1$ représente un atome d'hydrogène, un radical alkyle en $C_1$-$C_3$ ou un radical cycloalkyle en $C_3$-$C_6$ et encore plus préférentiellement parmi ceux dans lesquels $R_1$ représente un atome d'hydrogène, un radical éthyle ou cyclopropyle, étant entendu que parmi ces dernières désignations, la désignation éthyle est tout particulièrement préférée.

Selon cette même forme de réalisation de la description, le ou les composés de formule (I) sont de préférence choisis parmi ceux dans lesquels $R_2$ représente un radical alkyle en $C_1$-$C_3$ ou un radical cyclopropyle ; la désignation méthyle pour $R_2$ étant particulièrement préférée.

Par ailleurs, le ou les composés de formule (I) sont également de préférence choisis parmi ceux dans lesquels $R_3$ représente un atome d'hydrogène.

Selon une forme de réalisation particulièrement préférée de la description, la composition pharmaceutique comprend, à titre de composé de formule (I) particulier, de la (E)-N-(6-chloro-3-pyridylméthyl)-N-éthyl-N-méthyl-2-nitrovinylidènediamine, également dénommé nitenpyram.

Au sein de la composition pharmaceutique utilisée conformément à la description, le ou les composés de formule (I) représentent de préférence de 0,1 à 90% en poids par rapport au poids total de la composition, plus préférentiellement de 1 à 50% en poids du poids total de la composition, encore préférentiellement de 5 à 20% en poids du poids total de la composition et encore plus préférentiellement d'environ 10% en poids du poids total de la composition. Il doit être bien entendu toutefois que ces pourcentages peuvent être modulés selon les besoins de la formulation eu égard aux doses efficaces du principe actif considéré.

Selon la description, le ou les composés de formule (I) sont généralement administrés à l'animal en une dose unitaire comprise entre environ 0,01 et environ 800 mg/kg de poids corporel, plus préférentiellement entre environ 0,1 et 200 mg/kg de poids corporel, et encore plus préférentiellement entre environ 0,5 et 30 mg/kg de poids corporel.

Au sens de la présente description, on entend par "support liquide pharmaceutiquement acceptable", tout excipient liquide dont l'utilisation est compatible avec une administration topique chez les animaux domestiques, et en particulier chez le chat et le chien.

Selon la description, le support liquide pharmaceutiquement acceptable est de préférence un solvant tel qu'un solvant choisi parmi l'eau, un alcool, un éther de glycol ou un monoalkyléther de glycol et leur mélange. De préférence, l'alcool est choisi parmi l'éthanol et l'alcool benzylique ; l'éther de glycol est choisi parmi le n-butyléther du dipropylèneglycol et le propylène glycol ; et le monoalkyléther de glycol est choisi parmi le monométhyléther du propylèneglycol, le monométhyléther de l'éthylèneglycol, le monoéthyléther de l'éthylèneglycol et le monoéthyléther du diéthylène glycol.

D'autres solvants peuvent être utilisés à titre de support liquide pharmaceutiquement acceptable sans qu'il ait été observé de différence significative en termes d'efficacité de la composition. Ces solvants peuvent être choisis parmi les solvants organiques polaires ou non (apolaire), tels que le diméthylsulfoxyde (DMSO), le propylène carbonate, le N-méthyl-2-pyrrolidone (NMP), l'isopropylène glycérol, le N-octyl-pyrrolidone. La composition pharmaceutique peut aussi comporter des corps gras tels que les acides gras ou les esters d'acides gras en tant que solvants/cosolvants. A titre de corps gras, on peut citer en particulier l'acide oléique ou encore l'isopropyle myristate.

La composition pharmaceutique utilisée conformément à la description peut en outre renfermer un ou plusieurs excipients ayant différentes propriétés et pouvant par exemple être choisis parmi les agents tensioactifs tels que les polysorbates, notamment le polysorbate 80, les esters d'acides gras et d'alcools gras, notamment l'octanoate de cétostéaryle, les agents épaississants, les régulateurs de pH, les colorants, les arômes ou parfums, les antioxydants parmi lesquels on peut citer à titre d'exemple non limitatif le butylhydroxyanisole, le butylhydroxytoluène, le gallate de propyle, le palmitate d'ascorbyle, les extraits de romarin et leurs mélanges. En particulier, certains excipients peuvent contribuer à encore améliorer l'effet recherché de rémanence de l'activité contre les ectoparasites. Les inhibiteurs de cristallisation ainsi que les agents filmogènes tels que les polyvinylpyrrolidones et leurs copolymères sont des constituants de choix à cette fin.

Selon une forme de réalisation avantageuse de la description, outre le ou les composés de formule (I), la composition pharmaceutique comprend également un ou plusieurs composés actifs additionnels choisis parmi les principes actifs vis-à-vis de la prévention et/ou du traitement des puces et/ou des tiques et/ou des phlébotomes et des ectoparasites en général chez les animaux domestiques.

Parmi ces principes actifs additionnels, on peut tout particulièrement citer les dérivés de phénylpyrazoles tels que par exemple le fipronil, les pyréthrinoïdes tels que par exemple la fluméthrine et la perméthrine, les chloronicotinyles tels que par exemple l'imidaclopride, et les régulateurs de la croissance des insectes tels que le pyriproxyfène ou encore le (S)-méthoprène. De telles combinaisons d'actifs au sein de la composition peuvent être utiles en vue d'améliorer l'efficacité de chaque actif pris individuellement ou d'élargir le spectre d'action de la composition afin, par exemple, que la composition soit aussi efficace contre les infestations par les tiques et/ou les phlébotomes, ou bien en vue de diminuer les doses des actifs individuels pour lutter, par exemple, contre leurs éventuels effets négatifs dans l'environnement ou contre les phénomènes de résistance, tout en conservant une efficacité insecticide satisfaisante.

Pour la mise en oeuvre de l'utilisation selon la description, il est par ailleurs avantageux d'associer les composés de formule générale (I) à d'autres types de composés actifs additionnels choisis parmi les principes actifs vis-à-vis de la prévention et/ou du traitement des endoparasites en général chez les animaux domestiques tels que le praziquantel ou les avermectines et leurs dérivés comme par exemple l'ivermectine, l'abamectine, la doramectine ou encore les milbémycines telles que la milbémycine oxyme ou la moxidectine, afin de prévenir et/ou de traiter les parasitoses internes en même temps que les infestations externes.

Le choix de l'actif additionnel, lorsqu'il doit passer la barrière cutanée pour exercer une activité systémique, conditionnera le choix des excipients de la formulation. Cette formulation doit par ailleurs présenter une stabilité satisfaisante.

Selon une forme de réalisation particulièrement avantageuse, la composition pharmaceutique liquide renferme une association de (E)-N-(6-chloro-3-pyridylméthyl)-N-éthyl-N'-méthyl-2-nitrovinylidènediamine (nitenpyram) et de 5-amino-1-[2,6-dichloro-4-(trifluorométhyl)phényl]-4-(trifluorométhylsulfinyl)-1H-pyrazole-3-carbonitrile (fipronil). Les Inventeurs ont en effet démontré que l'administration topique de nitenpyram seul présente déjà une efficacité supérieure à celle du fipronil, qui est le produit actuellement considéré comme le plus efficace du marché, mais que l'efficacité du nitenpyram peut encore être améliorée lorsqu'on l'administre de façon concomitante avec du fipronil.

Le rapport pondéral nitenpyram/fipronil est de préférence compris entre 1/10 et 10/1. Dans une forme de réalisation particulièrement préférée de la description, ce rapport pondéral est de 1/1.

Une composition pharmaceutique particulièrement préférée selon la description comprend (en poids par rapport au poids total de ladite composition) :

- 1 à 20 % de nitenpyram,
- 1 à 20 % de fipronil,
- 98 à 60 % d'un ou de plusieurs excipients tels que définis précédemment.

Selon une autre forme de réalisation avantageuse, la composition pharmaceutique renferme une association de (E)-N-(6-chloro-3-pyridylméthyl)-N-éthyl-N'-méthyl-2-nitrovinylidènediamine (nitenpyram) et de N,N'-[(méthylimino)-diméthylidyne]di-2,4-xylidine (amitraz).

Selon une autre forme de réalisation avantageuse, la composition pharmaceutique renferme une association de Nitenpyram, de Moxidectine et de Praziquantel.

Selon encore une autre variante, la composition pharmaceutique renferme une association de (E)-N-(6-chloro-3-pyridylméthyl)-N-éthyl-N'-méthyl-2-nitrovinylidènediamine (nitenpyram) et de 3-phénoxybenzyl (1RS)-cis,trans-3-(2,2-dichlorovinyl)-2,2-diméthylcyclopropanecarboxylate (perméthrine). La composition peut alors se présenter sous forme d'une suspension ou d'une solution pour application topique ou encore sous la forme d'un dispositif pour relargage contrôlé des principes actifs sur la peau et les poils de l'animal domestique tel qu'un collier, par exemple.

La composition pharmaceutique liquide utilisée conformément à la description peut facilement être préparée par simple dilution ou dissolution du ou des composés de formule (I) et éventuellement du ou des principes actifs additionnels dans le ou les solvants utilisés.

Après sa préparation, la composition pharmaceutique liquide est de préférence conditionnée en pipettes monodose.

Selon la description, la composition pharmaceutique liquide est de préférence administrée par application directe sur la peau de l'animal, au niveau des omoplates ou sur une ligne dorsale partant de la base de la queue et remontant jusqu'au cou.

Selon un autre de ses objets, la présente description se rapporte au composé de formule (I) pour son utilisation pour la prévention et/ou le traitement des infestations par les ectoparasites, en particulier par les puces, des animaux domestiques, en particulier le chien et le chat. La méthode consiste alors essentiellement en l'administration topique de dérivés de formule (I) au moins toutes les 2 semaines.

Outre les dispositions qui précèdent, la description comprend encore d'autres dispositions qui ressortiront de la description qui va suivre, qui se réfère à des exemples mettant en évidence l'efficacité du nitenpyram, lorsqu'il est administré seul ou en association avec du fipronil par application topique, ainsi qu'aux figures 1 à 5 qui représentent l'efficacité (en %) comparative de différents traitements antipuces (nitenpyram seul à 10 % : carrés pleins; nitenpyram à 10 % + fipronil à 10% : losanges pleins ; fipronil seul à 10 % : triangles pleins) chez le chat en fonction du temps (en jours).

Il doit être bien entendu toutefois que ces exemples sont donnés uniquement à titre d'illustration de l'objet de la description, dont ils ne constituent en aucune manière une limitation.

## EXEMPLE 1 : ÉTUDE DE LA RAPIDITÉ D'ACTION D'UNE COMPOSITION A BASE DE NITENPYRAM ADMINISTRÉE PAR VOIE TOPIQUE

Dans cet exemple, on a réalisé une étude comparative de l'efficacité du nitenpyram (rapidité d'action) administré par voie topique, seul ou en association avec du fipronil par rapport au fipronil administré seul, contre les puces du chat.

**1) Matériel et Méthodes**

a) Type d'étude

Il s'agit d'une étude d'efficacité contrôlée en aveugle, randomisée, réalisée en parallèle sur 4 groupes de six chats, selon les recommandations des Directives du Comité sur les produits médicinaux vétérinaires ("Committee for Veterinary Medicinal Products" (CVMP) : "*Guidelines for the testing and evaluation of the efficacy of anti parasitic substances for the treatment and prevention of tick and flea infestations in dogs and cats*", EMEA/CVMP/005/00-FINAL-Rev.1).

b) Animaux utilisés et conditions de maintenance

Les chats utilisés dans cette étude étaient des chats domestiques adultes, males ou femelles, âgés de plus de 6 mois, de races mélangées, mais principalement de race européenne à poils courts, pesant entre 2 et 5 kg. Avant le début de l'étude, il a été vérifié que tous les chats étaient en bonne santé, qu'ils n'étaient pas infestés de puces et que les femelles n'étaient pas enceintes. Tous les chats ont été vermifugés et acclimatés aux conditions de vie pendant au moins 7 jours avant le démarrage de l'étude.

Il a également été vérifié que les chats n'avaient reçu aucun traitement topique contre les puces au cours de 12 semaines précédent le début de l'étude.

Pendant la période d'acclimatation et toute la durée de l'étude, les chats ont été gardés à l'intérieur dans une pièce climatisée, chaque chat étant confiné dans des cages individuelles en acier inoxydable de dimensions 70 x 60 x 75 cm sans litière. Le numéro d'identification, le n° de groupe et le type de composition administrée a été noté sur chaque cage. La température de la pièce a été maintenue à une température d'environ 20°C ± 4 °C. Les chats ont été soumis à une alternance de 12 heures de lumière et de 12 heures d'obscurité.

Les animaux ont été nourris une fois par jour avec des croquettes pour chat du commerce, vendues sous la marque Iams® Adult selon les recommandations du fabricant, et ils ont eu de l'eau potable fraîche à volonté.

c) Compositions testées

On a préparé les compositions suivantes, dont la formulation est reportée dans le Tableau I ci-après :

**TABLEAU I**

| Ingrédients | COMPOSITIONS | | |
|---|---|---|---|
| | A | B | C[a] |
| Nitenpyram (% p/v) | 10 | 10 | - |
| Fipronil (% p/v) | 10 | - | 10 |
| Monoéthyléther du diethylène glycol | qs | qs | - |
| Excipients du produit Frontline® Top-Spot | - | - | Qs |
| [a] : la composition C correspond au produit commercial vendu sous la dénomination Frontline® Top-Spot par la société Mérial. Il a été utilisé tel que fourni par le fabricant. | | | |

d) Traitements

Groupe 1 : Traitement avec la composition A à raison de 0,5 ml par chat,
Groupe 2 : Traitement avec la composition B à raison de 0,5 ml par chat,
Groupe 3 : Traitement avec la composition C à raison de 0,5 ml par chat,
Groupe 4 : Contrôle négatif : pas de traitement.

Le traitement a été appliqué de façon topique, entre les omoplates des chats, en une seule fois au commencement de l'étude (J = 0).

e) Infestations par les puces / Mesure de l'efficacité des traitements

6 jours avant le commencement de l'étude (J = -6), tous les chats ont été infestés par environ 100 puces de laboratoire, souche *Ctenocephalis felis,* de sexe male ou femelle. Les puces ont ensuite été comptées la veille du traitement (J = -1) puis à différentes reprises après application du traitement (1, 4, 12 et 24 heures après le traitement).
Pour ce faire, toutes les puces présentes sur un animal sont récoltées par peignage du chat puis comptées après peignage. Le nombre de puces vivantes est ainsi déterminé. Après chaque comptage, les puces vivantes sont remises sur le chat.
A chaque comptage, l'efficacité du traitement a été calculée selon l'équation suivante :

$$\% \text{ d'efficacité} = 100 \text{ x } (NP_vC - NP_vT) / NP_vC$$

dans laquelle :

- $NP_vC$ est la moyenne géométrique du nombre de puces vivantes comptées sur les chats du groupe 4 (contrôle) ;
- $NP_vT$ est la moyenne géométrique du nombre de puces vivantes comptées sur les chats d'un groupe ayant reçu un traitement (Groupes 1, 2 ou 3).

**2) Résultats**

Les résultats obtenus sont représentés sur la figure 1 annexée sur laquelle le pourcentage d'efficacité de chaque traitement est fonction du temps (en heures).
Sur cette figure, les losanges pleins correspondent aux résultats du groupe 1 (Traitement avec la composition A contenant 10 % de nitenpyram et 10 % de fipronil), les carrés pleins correspondent aux résultats du groupe 2 (Traitement avec la composition B contenant 10 % de nitenpyram) et les triangles pleins correspondent aux résultats du groupe 3 (Traitement avec la composition C contenant 10 % de fipronil).
Ces résultats montrent que l'administration du nitenpyram par voie topique à un effet léthal sur les puces du chat. Ces résultats montrent également que les compositions A et B conformes à la présente description, c'est-à-dire comprenant du nitenpyram, ont une action plus rapide que la composition C de l'art antérieur à base de fipronil. Cette supériorité est encore plus marquée dans le cas de la composition A qui contient une association de nitenpyram et de fipronil.

**EXEMPLE 2 : ÉTUDE PRELIMINAIRE DE L'EFFICACITÉ DANS LE TEMPS D'UNE COMPOSITION A BASE DE NITENPYRAM CONTRE LES PUCES CHEZ LE CHAT**

Dans cet exemple, on a étudié la durée d'efficacité d'une formulation topique contenant 20 % en poids de nitenpyram contre les infestations par les puces chez le chat.
Cette étude a été réalisée sur deux chats de race européenne pesant respectivement 5,5 et 3,8 kg. Pendant toute la durée de l'expérience, les chats ont été placés en cage et nourris selon le protocole indiqué ci-dessus à l'exemple 1.
Un jour avant l'administration du traitement, les chats ont été infestés par une quantité connue de puces de race identique à celle utilisée dans l'exemple 1 ci-dessus.
Chaque chat a ensuite reçu, entre les omoplates, une dose de 0,5 ml d'une formulation topique ayant la composition suivante :

- nitenpyram 20 g
- Monoéthyléther du diethylène glycol qs 100 ml

Il a ensuite été procédé au comptage du nombre de puces encore vivantes 4 heures après l'administration de la composition (J = 0).
Les chats ont à nouveau été infestés par une quantité connue de puces 7 jours (J = 7), 14 jours (J = 14), 21 jours (J = 21), 35 jours (J = 35) et 42 jours (J = 42) après l'administration du traitement.
Un comptage des puces encore vivantes a alors été effectué 24 heures après chacune de ces nouvelles infestations (J = 8 ; J = 15; J = 22 ; J = 36 et J = 43).
Le pourcentage d'efficacité du traitement a alors été calculé en appliquant la formule suivante :

$$\% \text{ d'efficacité} = 100 \text{ x } (\text{NPinit} - \text{NPV}) / \text{NPinit.}$$

dans laquelle :

- NPinit. correspond au nombre de puces initial avant administration du traitement,
- NPV : correspond au nombre de puces encore vivantes à J = 0, J = 8 ; J = 15 ; J = 22 ; J = 36 ou J = 43.

Les résultats moyens obtenus sont reportés dans le tableau II ci après :

**TABLEAU II**

| | J = 0 | J = 8 | J = 15 | J = 21 | J = 36 | J = 43 |
|---|---|---|---|---|---|---|
| **Nombre de puces vivantes (moyenne des deux chats)** | 0 | 0 | 0 | 0 | 0 | 2,9 |
| **Efficacité (%)** | 100 | 100 | 100 | 100 | 100 | 92,8 |

Ces résultats démontrent que l'application unique d'une composition topique à base de nitenpyram a une efficacité prolongée dans le temps, puisque 35 jours après l'administration de la composition insecticide, on observe encore 100 % d'efficacité (effet rémanent).

**EXEMPLE 3 : ÉTUDE COMPLEMENTAIRE DE L'EFFICACITÉ DANS LE TEMPS D'UNE COMPOSITION A BASE DE NITENPYRAM CONTRE LES PUCES CHEZ LE CHAT**

Dans cet exemple, on a réalisé une étude comparative de l'efficacité, au cours du temps, du nitenpyram administré par voie topique, seul ou en association avec du fipronil par rapport au fipronil administré seul, contre les puces du chat. Il s'agit de la poursuite de l'étude de l'exemple 1 utilisant les compositions A, B et C.

**1) Mesure de l'efficacité des traitements**

A intervalle régulier après la première administration (J = 0), tous les chats ont été infestés par environ 100 puces de laboratoire, souche *Ctenocephalis felis,* de sexe male ou femelle. Les ré-infestations ont ainsi eut lieu à J = 7, J = 14, J = 21, J = 28, J = 35, J = 42, J = 49, J = 56 et J = 63. Les puces ont ensuite été comptées à différentes reprises après application du traitement (à 2, 4 et 12 heures après réinfestation à J = 7, J = 14, J = 21 et J = 28 ; à 4, 8 et 12 heures après réinfestation à J = 35 et J = 42 ; à 12, 24 et 48 heures après réinfestation à J = 49 ; à 24 et 48 heures après réinfestation à J = 56 et enfin à 48 heures après réinfestation à J = 63).

Pour ce faire, toutes les puces présentes sur un animal sont récoltées par peignage du chat puis comptées après peignage. Le nombre de puces vivantes est ainsi déterminé. Après chaque comptage, les puces vivantes sont remises sur le chat.

A chaque comptage, l'efficacité du traitement a été calculée selon l'équation suivante :

$$\% \text{ d'efficacité} = 100 \text{ x } (NP_vC - NP_vT) / NP_vC$$

dans laquelle :

- $NP_vC$ est la moyenne géométrique du nombre de puces vivantes comptées sur les chats du groupe 4 (contrôle) ;
- $NP_vT$ est la moyenne géométrique du nombre de puces vivantes comptées sur les chats d'un groupe ayant reçu un traitement (Groupes 1, 2 ou 3).

**2) Résultats**

Les résultats obtenus sont représentés dans le tableau III ci-après qui représente, pour chaque groupe, le pourcentage de puces mortes comptabilisées à différents temps après l'infestation :

**TABLEAU III**

| Jours de l'infestation | Temps (heures après l'infestation) | GROUPE 1 (Fipronil 10% + Nitenpyram 10%) | GROUPE 2 (Nitenpyram 10 %) | GROUPE 3 (Fipronil 10%) |
|---|---|---|---|---|
| J = 7 | 2 | 68,6 | 82,1 | 51,0 |
| | 4 | 99,2 | 99,8 | 96,8 |
| | 12 | 100,0 | 100,0 | 100,0 |
| J = 14 | 2 | 54,1 | 58,1 | 50,2 |
| | 4 | 95,8 | 98,4 | 96,2 |
| | 12 | 100,0 | 100,0 | 100,0 |
| J = 21 | 2 | 46,9 | 66,9 | 21,7 |
| | 4 | 93,1 | 97,5 | 87,5 |
| | 12 | 99,4 | 100,0 | 100,0 |
| J = 28 | 2 | 27,4 | 32,8 | 18,6 |
| | 4 | 71,4 | 85,1 | 80,0 |
| | 12 | 99,4 | 99,7 | 100,0 |
| J = 35 | 4 | 61,8 | 55,0 | 34,4 |
| | 8 | 89,9 | 95,4 | 83,4 |
| | 12 | 96,5 | 99,4 | 93,2 |
| J = 42 | 8 | 45,6 | 48,1 | 36,9 |
| | 12 | 83,3 | 93,1 | 78,2 |
| | 24 | 99,3 | 100,0 | 91,7 |
| J = 49 | 12 | 65,0 | 65,5 | 45,7 |
| | 24 | 95,2 | 96,6 | 84,5 |
| | 48 | 100,0 | 100,0 | 89,6 |
| J = 56 | 24 | 80,7 | 72,6 | 54,1 |
| | 48 | 98,2 | 94,4 | 69,9 |
| J = 61 | 48 | 70,0 | 69,6 | - |

Ces résultats sont également reportés sur les figures 2 à 5 annexées qui permettent de comparer à la fois la rapidité avec laquelle les différents traitements testés sont efficaces et la rémanence de ces traitements appliqués de manière topique. On utilise ici la norme admise de 95% en termes d'efficacité insecticide : 95% des puces doivent être tuées pour que le traitement vétérinaire soit considéré comme efficace.

La figure 2 représente l'efficacité comparative des trois compositions A, B et C (en %), mesurée par comptage des puces, en fonction du temps, c'est-à-dire 4 heures après la réinfestation les jours J = 7 ; J = 14 ; J = 21 ; J = 28 ; J = 35 ; J = 42 et J = 49.

La figure 3 représente l'efficacité comparative des trois compositions A, B et C (en %), mesurée par comptage des puces, en fonction du temps, c'est-à-dire 12 heures après la réinfestation les jours J = 7 ; J = 14 ; J = 21 ; J = 28 ; J = 35 ; J = 42 et J = 49.

La figure 4 représente l'efficacité comparative des trois compositions A, B et C (en %), mesurée par comptage des puces, en fonction du temps, c'est-à-dire 24 heures après la réinfestation les jours J = 7 ; J = 14 ; J = 21 ; J = 28 ; J = 35 ; J = 42 ; J = 49 et J = 56.

La figure 5 représente l'efficacité comparative des trois compositions A, B et C (en %), mesurée par comptage des puces, en fonction du temps, c'est-à-dire 48 heures après la réinfestation les jours J = 7 ; J = 14 ; J = 21 ; J = 28 ; J = 35 ; J = 42 ; J = 49 ; J = 56 et J = 61.

Sur toutes ces figures, les carrés pleins correspondent aux résultats du groupe 1 (Traitement avec la composition A contenant 10 % de nitenpyram et 10 % de fipronil), les ronds pleins correspondent aux résultats du groupe 2 (Traitement avec la composition B contenant 10 % de nitenpyram) et les triangles pleins correspondent aux résultats du groupe 3 (Traitement avec la composition C contenant 10 % de fipronil).

Ces résultats montrent que la période de la protection résiduelle, calculée en nombre de semaines, pendant laquelle l'efficacité des compositions contre les puces est supérieure ou égale à 95% est de :

- Quatre semaines pour le Groupe 3 (Traitement avec la composition C contenant 10 % de fipronil seul) quand elle est évaluée jusqu'à 12 heures après la réinfestation ;
- Sept semaines pour le Groupe 2 (Traitement avec la composition B contenant 10 % de nitenpyram seul) quand elle est évaluée jusqu'à 48 heures après la réinfestation ; et
- Huit semaines pour le Groupe 1 (Traitement avec la composition A contenant 10 % de nitenpyram et 10 % de fipronil) quand elle est évaluée 48 heures après la réinfestation.

Ces résultats montrent que l'administration du nitenpyram par voie topique à un effet létal rapide et prolongé sur les puces du chat. Ces résultats montrent également que les compositions A et B conformes à la présente description, c'est-à-dire comprenant du nitenpyram, ont une action plus rapide que la composition C de l'art antérieur à base de fipronil seul. Cette supériorité est encore plus marquée dans le cas de la composition A qui contient une association de nitenpyram et de fipronil.

Ces résultats confirment par ailleurs que l'application unique d'une composition topique à base de nitenpyram a une efficacité prolongée dans le temps, puisque 57 jours après l'administration de la composition insecticide, on observe encore une efficacité contre les puces supérieure à 95%. L'effet rémanent de la composition à base de nitenpyram pour application topique est ainsi clairement démontré.

## EXEMPLE 4 : ÉTUDE COMPARATIVE DE LA PHARMACOCINETIQUE

## DU NITENPYRAM ADMINISTRE PAR VOIE ORALE ET TOPIQUE

Dans cet exemple, on a réalisé une étude comparative de la pharmacocinétique du nitenpyram administré par voie topique et par voie orale chez le chat.

Les chats utilisés dans cette étude ont été préparés dans les mêmes conditions que celles décrites dans l'exemple 1.

Il a été vérifié au préalable qu'aucune trace de nitenpyram n'était détectée dans le plasma des chats.

### 1) Matériel et Méthodes

Le profil pharmacocinétique du nitenpyram a été étudié selon les modalités suivantes.

Dix jeunes chats adultes pesant entre 2 et 6 kg sont répartis en deux groupes :

- Un premier groupe de 5 chats (femelles) a reçu une administration topique unique effectuée sur le dos entre les omoplates au commencement de l'étude ; la composition topique est une solution à 10% de nitenpyram (100 mg de nitenpyram par ml) et la quantité administrée est de 0,5 ml par chat.
- Un second groupe (4 femelles et un mâle) a reçu une administration orale unique du produit commercial Capstar® effectuée au commencement de l'étude. Le produit Capstar® est un comprimé comprenant 11,4 mg de nitenpyram.

Des échantillons de sang sont prélevés régulièrement, centrifugés à 3000 rpm pendant 10 minutes à 4°C, le culot est éliminé et le plasma est récupéré pour analyse.

La concentration plasmatique en nitenpyram est déterminée par la méthode UPLC-MS/MS (Ultra-Performance Liquid Chromatography coupled to tandem mass spectrometry) sur une colonne ACQUITY BEH $C_{18}$ et un spectophotomètre de masse Waters Quattro Premier XE.

### 2) Résultats

Il est constaté que l'administration topique de nitenpyram est bien tolérée localement. De même, l'administration orale du nitenpyram est bien tolérée.

Les paramètres pharmacocinétiques déterminés dans le cadre de la présente étude sont résumés dans le Tableau IV ci-après :

**TABLEAU IV**

| | Administration topique | Administration orale |
|---|---|---|
| **Cmax :** Quantité maximale de nitenpyram dans le plasma ($ng.ml^{-1}$) | 1291 | 5898 |
| **Tmax** : temps auquel est observée la concentration maximale en nitenpyram dans le plasma (h) | 15,0 | 1,3 |
| **AUCtot :** aire sous la courbe de la concentration en nitenpyram au cours du temps déterminée entre t0 et la dernière mesure ($ng.h.ml^{-1}$) | 86361 | 76773 |
| **Thalf :** temps de demi-vie (h) | 69,1 | 9,8 |

On observe que, lors de l'administration topique du nitenpyram, la concentration plasmatique maximale en actif (Cmax) est atteinte plus tardivement que lors de l'administration orale : le Tmax du nitenpyram topique étant de 15,0 heures contre 1,3 heure pour l'administration orale.

Bien que la concentration plasmatique maximale de nitenpyram soit plus faible lors de l'administration topique (1291 $ng.ml^{-1}$) que celle mesurée lors de l'administration orale (plus de 5800 $ng.ml^{-1}$), il est observé que la valeur de l'AUCtot, qui représente la quantité totale d'actif présente dans le plasma au long de l'expérimentation, est supérieure dans le cas de l'administration topique. En outre, le temps de demi-vie du nitenpyram (Thalf qui correspond à la moitié du temps nécessaire à l'élimination de l'actif) administré topiquement est plus de 7 fois plus long (69,1 heures) que celui du nitenpyram administré oralement (9,8 heures).

**3) Conclusion**

Ces résultats montrent que l'administration du nitenpyram par voie topique conduit au passage de l'actif dans le sang des animaux. Ce passage se fait de façon plus lente et plus longue que lors de l'administration orale du nitenpyram ; ceci permet d'expliquer en partie que le nitenpyram administré topiquement présente une efficacité prolongée dans le temps.

Ainsi, l'administration topique du nitenpyram offre l'avantage de présenter :

- un effet anti-puces immédiat par contact ; cet effet immédiat est nécessaire pour éviter toute piqûre de puce et la reproduction de la puce. On constate que l'efficacité par contact se prolonge dans le temps (voir exemples 2 et 3) ; et
- un effet systémique prolongé dans le temps (empoisonnement des puces par le sang) comparativement à celui obtenu lors de l'administration orale du nitenpyram.

L'effet de contact rémanent et l'effet systémique prolongé permettent une administration espacée de la composition et une posologie plus adaptée aux attentes des consommateurs.

**EXEMPLE 5 : FORMULATIONS TOPIQUES**

**Composition 1**

| | |
|---|---|
| Moxidectine | 2,5% |
| Praziquantel | 8,5% |
| Nitenpyram | 10% |
| BHA | 0,094% |
| BHT | 0,047% |
| Alcool benzylique | 25% |
| DMSO | 20% |
| Polysorbate 80 | 5% |
| PVP K17 | 5% |
| Solketal (ou isopropylidène glycérol) | qs. |

**Composition 2**

| | |
|---|---|
| Moxidectine | 2,5% |
| Praziquantel | 8,5% |
| Nitenpyram | 10% |
| BHA | 0,094% |
| BHT | 0,047% |
| Alcool benzylique | 25% |
| NMP | 20% |
| Polysorbate 80 | 5% |
| PVP K17 | 5% |
| Acide oléique | 5% |
| Propylène carbonate | qs. |

**Composition 3**

| | |
|---|---|
| Moxidectine | 2,5% |
| Praziquantel | 8,5% |
| Nitenpyram | 10% |
| BHA | 0,094% |
| BHT | 0,047% |
| Alcool benzylique | 25% |
| Isopropyl myristate | 10% |
| Polysorbate 80 | 5% |
| PVP K17 | 5% |
| Solketal | qs. |

**Composition 4**

| | |
|---|---|
| Moxidectine | 2,5% |
| Nitenpyram | 5% |
| BHA | 0,094% |
| BHT | 0,047% |
| Alcool benzylique | 25% |
| NMP | 20% |
| Polysorbate 80 | 5% |
| PVP K17 | 5% |
| Acide oléique | 5% |
| Propylène carbonate | qs. |

**Composition 5**

| | |
|---|---|
| Nitenpyram | 5 % |
| BHA | 0,094% |
| BHT | 0,047% |
| Alcool benzylique | 25% |
| NMP | 20% |
| Polysorbate 80 | 5% |
| PVP K17 | 5% |
| Acide oléique | 5% |
| Propylène carbonate | qs. |

**Composition 6**

| | |
|---|---|
| Praziquantel | 8,5% |
| Nitenpyram | 10% |
| BHA | 0,094% |
| BHT | 0,047% |
| Alcool benzylique | 25% |
| NMP | 20% |
| Polysorbate 80 | 5% |
| PVP K17 | 5% |
| Acide oléique | 5% |
| Propylene carbonate | qs. |

**Composition 7**

| | |
|---|---|
| Nitenpyram | 5% |
| BHA | 0,094% |
| BHT | 0,047% |
| Alcool benzylique | 25% |
| NMP | 20% |
| Polysorbate 80 | 5% |
| PVP K17 | 5% |
| Acide oléique | 5% |
| Propylene carbonate | qs. |

**Composition 8**

| | |
|---|---|
| Moxidectine | 2,5% |
| Praziquantel | 8,5% |
| Nitenpyram | 10% |
| Pyriproxifen | 10% |
| BHA | 0,094% |
| BHT | 0,047% |
| Alcool benzylique | 25% |
| NMP | 20% |
| Polysorbate 80 | 5% |
| PVP K17 | 2,5% |
| Propylene carbonate | qs. |

**Composition 9**

| | |
|---|---|
| Moxidectine | 2,5% |
| Praziquantel | 8,5% |
| Nitenpyram | 10% |
| Pyriproxifen | 10% |
| BHA | 0,094% |
| BHT | 0,047% |
| Alcool benzylique | 25% |
| Isopropyl myristate | 8% |
| Polysorbate 80 | 5% |
| PVPK17 | 2,5% |
| Solketal | qs. |

**Composition 10**

| | |
|---|---|
| Moxidectine | 2,5% |
| Praziquantel | 8,5% |
| Nitenpyram | 2,5% |
| Pyriproxifen | 10% |
| BHA | 0,094% |
| BHT | 0,047% |
| Alcool benzylique | 25% |
| DMSO | 20% |
| Polysorbate 80 | 5% |
| PVP K17 | 2,5% |
| Solketal | qs. |

**Composition 11**

| | |
|---|---|
| Nitenpyram | 15% |
| NMP | qsp 100 ml |

**Composition 12**

| | |
|---|---|
| Nitenpyram | 15% |
| N octyl pyrrolidone | qsp 100 ml |

**Composition 13**

| | |
|---|---|
| Nitenpyram | 15% |
| DMSO | qsp 100 ml |

**Composition 14**

| | |
|---|---|
| Amitraz | 10%, |
| Nitenpyram | 15% |
| DMSO | qsp 100 ml |

**Composition 15**

| | |
|---|---|
| Amitraz | 10% |
| Nitenpyram | 15% |
| NMP | qsp 100 ml |

**Composition 16**

| | |
|---|---|
| Amitraz | 10%, |
| Nitenpyram | 15%, |
| isopropyl myristate | 25% |
| NMP | qsp 100 ml |

Pour chacune des compositions 1 à 16, il a été observé une stabilité satisfaisante après un stockage de 4 mois à 25°C ; ceci démontre une absence d'incompatibilité physique des constituants.

## Revendications

1. (E)-N-(6-chloro-3-pyridylméthyl)-N-éthyl-N'-méthyl-2-nitrovinylidènediamine (nitenpyram) pour son utilisation pour la prévention et/ou le traitement des infestations par les puces chez les animaux domestiques,
**caractérisé en ce que** ledit nitenpyram est formulé, dans une composition pharmaceutique liquide à administrer par voie topique, avec un support liquide pharmaceutiquement acceptable consistant en du monoéthyléther du diéthylène glycol,
et **en ce que** ladite composition est appliquée au moins toutes les 4 semaines, intervalle de temps pendant lequel l'animal n'est pas retraité.

2. (E)-N-(6-chloro-3-pyridylméthyl)-N-éthyl-N'-méthyl-2-nitrovinylidènediamine (nitenpyram) selon la revendication 1 pour son utilisation selon la revendication 1, **caractérisé par le fait que** ladite composition pharmaceutique est administrée chez le chat ou le chien.

3. (E)-N-(6-chloro-3-pyridylméthyl)-N-éthyl-N'-méthyl-2-nitrovinylidènediamine (nitenpyram) selon la revendication 1 pour son utilisation selon l'une quelconque des revendications précédentes, **caractérisé par le fait qu'**au sein de ladite composition pharmaceutique, le nitenpyram représente de 5 à 20% en poids par rapport au poids total de la composition.

4. (E)-N-(6-chloro-3-pyridylméthyl)-N-éthyl-N'-méthyl-2-nitrovinylidènediamine (nitenpyram) selon la revendication 1 pour son utilisation selon l'une quelconque des revendications précédentes, **caractérisé par le fait que** ladite composition pharmaceutique renferme en outre un ou plusieurs excipients choisis parmi les agents tensioactifs, les agents épaississants, les régulateurs de pH, les colorants, les inhibiteurs de cristallisation, les agents filmogènes, les antioxydants, et leurs mélanges.

5. (E)-N-(6-chloro-3-pyridylméthyl)-N-éthyl-N'-méthyl-2-nitrovinylidènediamine (nitenpyram) selon la revendication 1 pour son utilisation selon l'une quelconque des revendications précédentes, **caractérisé par le fait que** ladite composition pharmaceutique comprend également un ou plusieurs composés actifs additionnels choisis parmi les principes actifs vis-à-vis de la prévention et/ou du traitement des puces et/ou des tiques et/ou des phlébotomes chez les animaux domestiques.

6. (E)-N-(6-chloro-3-pyridylméthyl)-N-éthyl-N'-méthyl-2-nitrovinylidènediamine (nitenpyram) selon la revendication 1 pour son utilisation selon la revendication 5, **caractérisé par le fait que** ladite composition pharmaceutique renferme une association de (E)-N-(6-chloro-3-pyridylméthyl)-N-éthyl-N'-méthyl-2-nitrovinylidènediamine (nitenpyram) et de 5-amino-1-[2,6-dichloro-4-(trifluorométhyl)phényl]-4-(trifluorométhylsulfinyl)-1H-pyrazole-3-carbonitrile (fipronil).

7. (E)-N-(6-chloro-3-pyridylméthyl)-N-éthyl-N'-méthyl-2-nitrovinylidènediamine (nitenpyram) selon la revendication 1 pour son utilisation selon l'une quelconque des revendications 1 à 4, **caractérisé par le fait que** ladite composition pharmaceutique comprend également un ou plusieurs composés actifs additionnels choisis parmi les principes actifs vis-à-vis de la prévention et/ou du traitement des endoparasites chez les animaux domestiques.

8. (E)-N-(6-chloro-3-pyridylméthyl)-N-éthyl-N'-méthyl-2-nitrovinylidènediamine (nitenpyram) selon la revendication 1 pour son utilisation selon la revendication 7, **caractérisée par le fait que** ladite composition pharmaceutique renferme une association de Nitenpyram, de Moxidectine et de Praziquantel.

## Patentansprüche

1. (E)-N-(6-chloro-3-pyridylmethyl)-N-ethyl-N'-methyl-2-nitrovinylidendiamin (Nitenpyram) für seine Verwendung bei der Prävention und/oder der Behandlung des Befalls durch Flöhe bei Haustieren,
**dadurch gekennzeichnet, dass** das Nitenpyram in einer flüssigen pharmazeutischen Zusammensetzung zur Verabreichung auf topischem Weg mit einem flüssigen pharmazeutisch akzeptablen Träger formuliert ist, der aus Diethylenglykolmonoethylether besteht,
und dadurch, dass die Zusammensetzung mindestens alle 4 Wochen angewendet wird, dem Zeitintervall, in dem das Tier nicht erneut behandelt wird.

2. (E)-N-(6-chloro-3-pyridylmethyl)-N-ethyl-N'-methyl-2-nitrovinylidendiamin (Nitenpyram) nach Anspruch 1 für seine Verwendung nach Anspruch 1, **dadurch gekennzeichnet, dass** die pharmazeutische Zusammensetzung bei der

Katze oder beim Hund verabreicht wird.

3. (E)-N-(6-chloro-3-pyridylmethyl)-N-ethyl-N'-methyl-2-nitrovinylidendiamin (Nitenpyram) nach Anspruch 1 für seine Verwendung nach einem der vorhergehenden Ansprüche, **dadurch gekennzeichnet, dass** innerhalb der pharmazeutischen Zusammensetzung das Nitenpyram von 5 bis 20 Gew.% mit Bezug auf das Gesamtgewicht der Zusammensetzung darstellt.

4. (E)-N-(6-chloro-3-pyridylmethyl)-N-ethyl-N'-methyl-2-nitrovinylidendiamin (Nitenpyram) nach Anspruch 1 für seine Verwendung nach einem der vorhergehenden Ansprüche, **dadurch gekennzeichnet, dass** die pharmazeutische Zusammensetzung außerdem mehrere Trägerstoffe einschließt, ausgewählt aus den oberflächenaktiven Mitteln, den Verdickungsmitteln, den pH-Regulatoren, den Farbstoffen, den Kristallisationshemmern, den filmbildenden Mitteln, den Antioxidationsmitteln und ihren Mischungen.

5. (E)-N-(6-chloro-3-pyridylmethyl)-N-ethyl-N'-methyl-2-nitrovinylidendiamin (Nitenpyram) nach Anspruch 1 für seine Verwendung nach einem der vorhergehenden Ansprüche, **dadurch gekennzeichnet, dass** die pharmazeutische Zusammensetzung auch ein oder mehrere zusätzliche aktive Mittel umfasst, ausgewählt aus den Wirkstoffen für die Prävention und/oder die Behandlung der Flöhe und/oder der Zecken und/oder der Sandmücke bei den Haustieren.

6. (E)-N-(6-chloro-3-pyridylmethyl)-N-ethyl-N'-methyl-2-nitrovinylidendiamin (Nitenpyram) nach Anspruch 1 für seine Verwendung nach Anspruch 5, **dadurch gekennzeichnet, dass** die pharmazeutische Zusammensetzung einen Verbund von (E)-N-(6-chloro-3-pyridylmethyl)-N-ethyl-N'-methyl-2-nitrovinylidendiamin (Nitenpyram) und von 5-Amino-1-[2,6-dichloro-4-(trifluoromethyl)phenyl]-4-(trifluoromethylsulfinyl)-1-H-pyrazol-3-carbonitril (Fipronil) einschließt.

7. (E)-N-(6-chloro-3-pyridylmethyl)-N-ethyl-N'-methyl-2-nitrovinylidendiamin (Nitenpyram) nach Anspruch 1 für seine Verwendung nach einem der vorhergehenden Ansprüche 1 bis 4, **dadurch gekennzeichnet, dass** die pharmazeutische Zusammensetzung auch einen oder mehrere zusätzliche aktive Mittel umfasst, ausgewählt aus den Wirkstoffen für die Prävention und/oder die Behandlung der Endoparasiten bei den Haustieren.

8. (E)-N-(6-chloro-3-pyridylmethyl)-N-ethyl-N'-methyl-2-nitrovinylidendiamin (Nitenpyram) nach Anspruch 1 für seine Verwendung nach Anspruch 7, **dadurch gekennzeichnet, dass** die pharmazeutische Zusammensetzung einen Verbund aus Nitenpyram, Moxidectin und Praziquantel einschließt.

## Claims

1. (E)-N-(6-chloro-3-pyridylmethyl)-N-ethyl-N'-methyl-2-nitrovinylidenediamine (nitenpyram) for use thereof for the prevention and/or treatment of flea infestations in domestic animals,
**characterised in that** said nitenpyram is formulated, in a liquid pharmaceutical composition for topical administration, with a pharmaceutically acceptable liquid substrate consisting of diethylene glycol monoethyleether,
and **in that** said composition is applied at least every 4 weeks, during which time interval the animal is not retreated.

2. (E)-N-(6-chloro-3-pyridylmethyl)-N-ethyl-N'-methyl-2-nitrovinylidenediamine (nitenpyram) according to claim 1 for use thereof according to claim 1, **characterised in that** said pharmaceutical composition is administered to cats or dogs.

3. (E)-N-(6-chloro-3-pyridylmethyl)-N-ethyl-N'-methyl-2-nitrovinylidenediamine (nitenpyram) according to claim 1 for use thereof according to any one of the preceding claims, **characterised in that**, within said pharmaceutical composition, nitenpyram represents 5 to 20% by weight with respect to the total weight of the composition.

4. (E)-N-(6-chloro-3-pyridylmethyl)-N-ethyl-N'-methyl-2-nitrovinylidenediamine (nitenpyram) according to claim 1 for use thereof according to any one of the preceding claims, **characterised in that** said pharmaceutical composition further contains one or a plurality of excipients chosen from among surfactants, thickening agents, pH regulators, colorants, crystallisation inhibitors, film-forming agents, antioxidants, and mixtures thereof.

5. (E)-N-(6-chloro-3-pyridylmethyl)-N-ethyl-N'-methyl-2-nitrovinylidenediamine (nitenpyram) according to claim 1 for use thereof according to any one of the preceding claims, **characterised in that** said pharmaceutical composition

also comprises one or a plurality of additional active compounds chosen from among active ingredients with respect to the prevention and/or treatment of fleas and/or ticks and/or sand flies in domestic animals.

**6.** (E)-N-(6-chloro-3-pyridylmethyl)-N-ethyl-N'-methyl-2-nitrovinylidenediamine (nitenpyram) according to claim 1 for use thereof according to claim 5, **characterised in that** said pharmaceutical composition contains an association of (E)-N-(6-chloro-3-pyridylmethyl)-N-ethyl-N'-methyl-2-nitrovinylidenediamine (nitenpyram) and of 5-amino-1-[2,6-dichloro-4-(trifluoromethyl)phenyl]-4-(trifluoromethylsulphinyl)-1H-pyrazole-3-carbonitrile (fipronil).

**7.** (E)-N-(6-chloro-3-pyridylmethyl)-N-ethyl-N'-methyl-2-nitrovinylidenediamine (nitenpyram) according to claim 1 for use thereof according to any one of claims 1 to 4, **characterised in that** said pharmaceutical composition also comprises one or a plurality of additional active compounds chosen from among active ingredients with respect to the prevention and/or treatment of endoparasites in domestic animals.

**8.** (E)-N-(6-chloro-3-pyridylmethyl)-N-ethyl-N'-methyl-2-nitrovinylidenediamine (nitenpyram) according to claim 1 for use thereof according to claim 7, **characterised in that** said pharmaceutical composition contains an association of Nitenpyram, Moxidectin and Praziquantel.

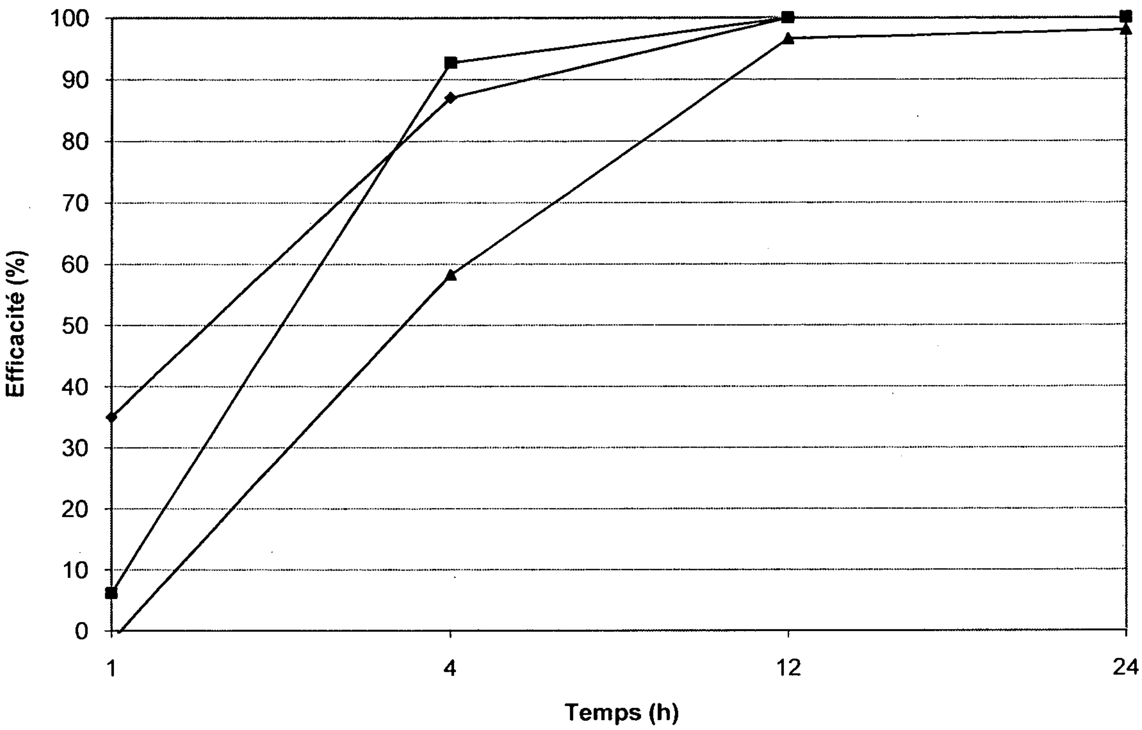


FIGURE 1

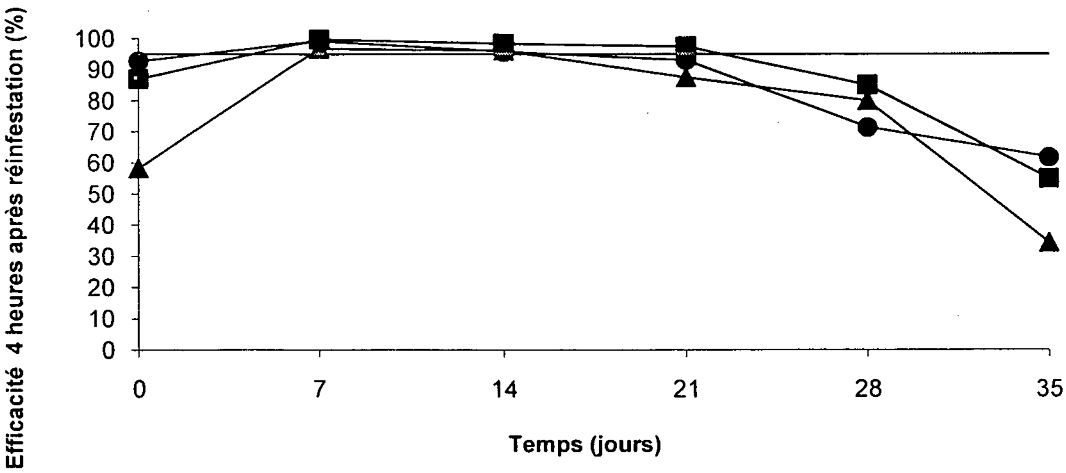


FIGURE 2

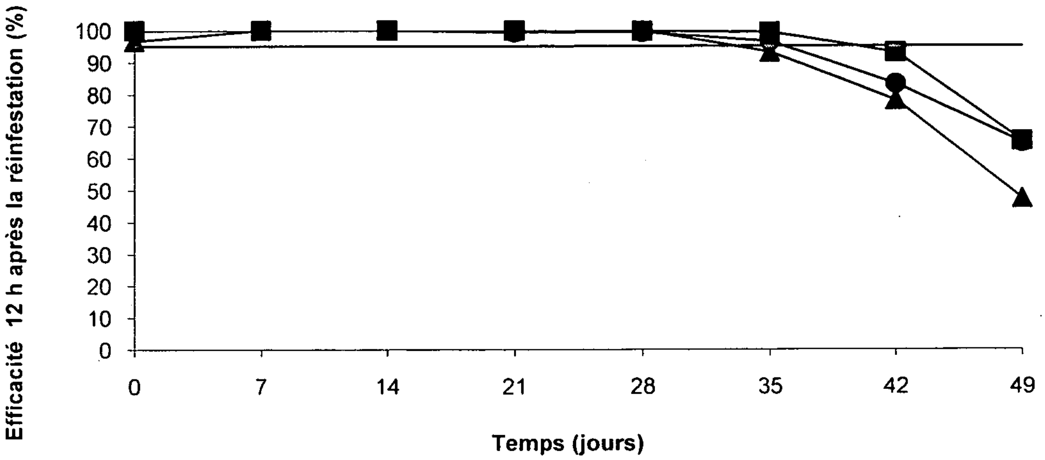


FIGURE 3

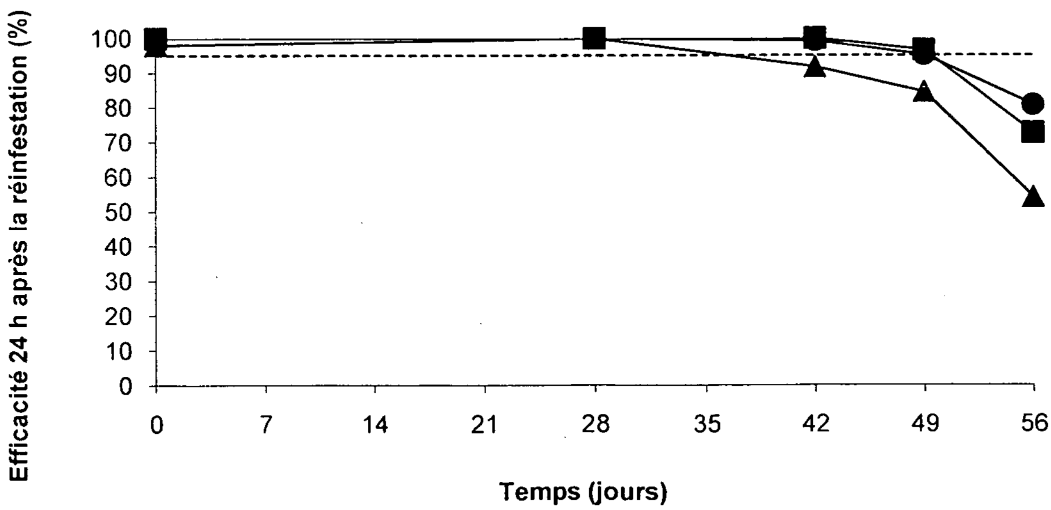


FIGURE 4

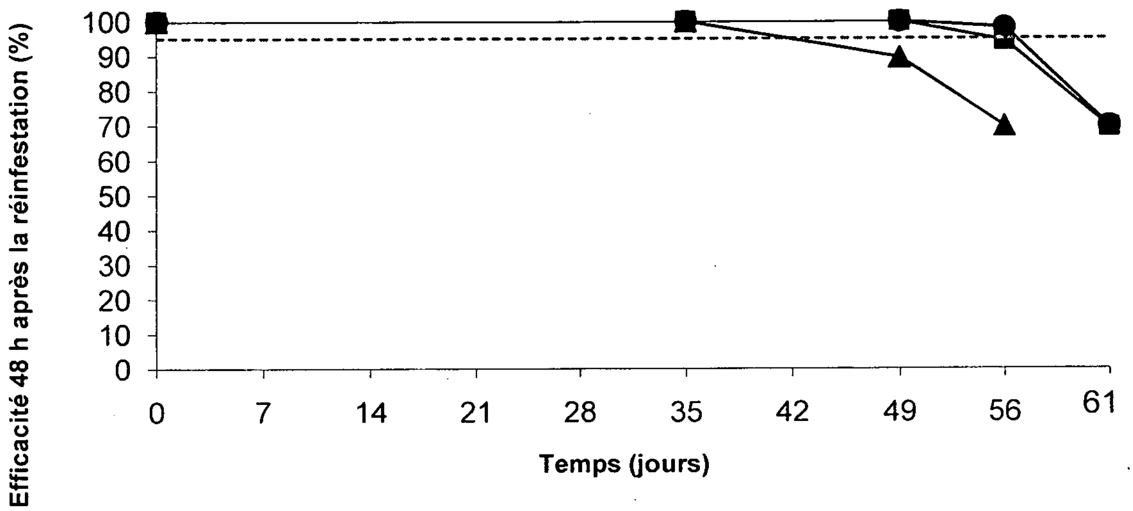
Efficacité 48 h après la réinfestation (%)
100
90
80
70
60
50
40
30
20
10
0
0
7
14
21
28
35
42
49
56
61
Temps (jours)

FIGURE 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0616494 A
- DE 19807633
- EP 0976328 A
- WO 2004064522 A
- EP 1668984 A
- JP 2001291535 A
- JP 2001216111 A
- WO 0029378 A